# EUROPEAN PATENT SPECIFICATION

(11) **EP 1 083 886 B1**
(45) Date of publication and mention of the grant of the patent: **02.04.2003**
(21) Application number: 99953285.6
(22) Date of filing: 27.05.1999
(51) Int. Cl.: A61K 9/20, A61K 9/00, A61K 9/14

(54) **DRUG DELIVERY SYSTEM COMPRISING A TIGHTLY COMPACTED SOLID MEDICAMENT STOCK**
ARZNEISTOFF-ABGABESYSTEM ENTHALTEND EINE FESTGEPACKTE, FESTE ARZNEISTOFFBASIS
SYSTEME DE PRESENTATION DE MEDICAMENT COMPRENANT DE LA MATIERE MEDICAMENTEUSE SOLIDE FORTEMENT COMPACTEE

(30) Priority: 27.05.1998 US 86895 P
(43) Date of publication of application: 21.03.2001
(73) Proprietor: Euroceltique S.A., Luxembourg (LU)
(72) Inventor: FLEISCHER, Wolfgang, D-55218 Ingelheim (DE); REIMER, Karen, D-65582 Hambach (DE); SCHREIER, Hans, 41460 Neuss (DE)
(74) Representative: Maiwald, Walter, Dr. Dipl.-Chem.
(86) International application number: EP9903680
(87) International publication number: WO99061003

(56) References cited:
- EP-A- 0 260 241
- EP-A- 0 407 028
- WO-A-94/14490
- WO-A-94/28876

## Description

This invention is concerned with a drug delivery system comprising a tightly compacted solid medicament stock, a method for the preparation of the medicament stock as well as the use of the solid medicament stock of the invention.

Pulmonary application by way of inhalation or insufflation of drug substances has e.g. been achieved by administration of the respective drug as an aerosol or by powder inhalation. Since aerosols are often based on fluorohydrocarbons or other hydrocarbons, it is generally desirable to substitute aerosols by powder inhalation.

EP 0 407 028 discloses a device for administering drugs by inhalation in powdered form. The drawback of the device and the medicament disclosed is that the solid reservoir cannot be handled safely since only low pressures are applied for compactation. The compacted body is brittle and may fall apart when not handled carefully.

WO 94/14490 and WO 93/29165 are concerned with a system for application of powder or fine particles via inhalation. The device according to WO 93/24165 allows generation of particles from a compacted tablet which has an isotropic substructure. The compacted medicament according to WO 94/14490 is manufactured by applying pressures of up to 500 MPa, thus some orders of magnitude higher than those applied in EP 0 407 028. The compacted body according to WO 94/14490 is advantageous as compared to the compacted body according to EP 0 407 028. It allows e.g. easy handling since caused by its tightness it does not fall apart. Furthermore, it does not tend to absorb water as compared to the less compacted body according to EP 0 407 028.

Liposomes are well known carriers of pharmaceutical compositions. Advantages of application of medicaments via liposomes have been subject of several reviews. Pulmonary application of liposomes has permitted major advances in the treatment of infectious diseases and asthma (compare e.g. H. Schreier, Pulmonary aplications of liposomes, in "Medical applications of liposomes", Paphadjopoulos & Lasic (eds.), Elsevier, 1997; RJ Gonzalez-Rothi & H. Schreier, Pulmonary delivery of liposome encapsulated drugs in asthma therapy, Clin. Immunother. 4, 331 - 337, 1995; H. Schreier, R.J. Gonzalez-Rothi. A. A. Stecenko, Pulmonary delivery of liposomes, J. Controlled Release 24, 209 - 223, 1993). The diseased lung is particularly accessible to topical therapy by inhalation of drug aerosols (H. Schreier and S. M. Sawyer, Liposomal DNA vectors for cystic fibrosis gene therapy, Current applications, limitations, and future directions, Adv. Drug. Del. Rev. 19, 73 - 87, 1996; L. Gagné & H. Schreier, Aerosolization of plasmid DNA. Protective effects of solute condensing agents, and liposome carriers. Proceed. Intern. Symp. Control. Rel. Bioact. Mater., 1997). While antiasthmatic and antiallergic agents delivered by metered dose inhalers (MDI's) are widely used, aerosolized antimicrobiols e.g. aminoglycosides for the management of cystic fibrosis, ribavirin for respiratory syncytial virus infections in infants and pentamidine for the treatment of pulmonary Pneumocystis carinii infections in immuno-compromised patients have only recently been introduced (WO 96/27393, H. Schreier, K.J. McNicol, M. Ausborn, D.W. Soucy, H. Derendorf, A.A. Stecenko, R.J. Gonzalez-Rothi, Pulmonary delivery of amikacin liposomes and acute liposome toxicity in the sheep. Int. J. Pharmaceut. 87, 183 - 193, 1992).

Other particulate carriers have been described in the art, which are comparable to liposomes. These include microspheres, nanoparticles. Large Porous Particles, pulse laser polymer coated molecules, artificial virus envelopes etc.

Where alternative particulate carriers are used, they are generally prepared as known in the art. Thus, microspheres which are used to deliver a very wide range of therapeutic or cosmetic agents, are made as described for example in WO 95/15118.

Nanoparticles may in some cases be used, provided that they can be loaded with a sufficient amount of active agent and can be administered to the lower respiratory tract according to this invention. They can be prepared according to the methods known in the art, as e.g. described by Heyder (GSF München) in "Drugs delivered to the lung", Abstracts IV, Hilton Head Island Conference, May 1998.

Methods using a pulse laser deposition (PLD) apparatus and a polymeric target to apply coatings to drug powders in a short non-aqueous process are also suitable for the formation of particulate preparations according to this invention. These have e.g. been described by Talton et al., "Novel Coating Method for Improved Dry Delivery", Univ. of Florida UF 1887 (1998).

A further suitable delivery system employs Large Porous Particles as disclosed by David A. Edwards et al. in " Large Porous Particles for Pulmonary Drug Delivery" (Science, 20: June 1997, Vol. 276, p. 1868-1871).

Where hereinafter reference is made to liposomes, it should be understood that the invention may be operated using suitable such alternative particulate carriers combined with, or instead of, liposomes.

Pulmonary delivery of drugs is complicated by (i) the need for training patients to coordinate breathing and inhaling of aerosols, (ii) rapid absorption of most drugs, necessitating frequent dosing which often is responsible for systemic side effects, (iii) poor aqueous solubility of drugs which may cause local irritation and inflammation in the airways or prevent the use of aerosols entirely, and (iv) poor cytosolic penetration of drug to treat intracellular pathogens. Illustrative of such problems is the controversial use of ribavirin aerosols which requires excessive aerosolization ("aerosol tent"), frequent monitoring of valves, tubing changes. and endotracheal tube suctioning to prevent precipitation of drug, while the clinical result is modest at best. Another therapeutically undesirable aspect of pulmonary drug delivery is rapid absorption of most drugs from the lung, necessitating frequent dosing, e.g. of bronchodilators and corticosteroids. Particulate carriers, especially liposomes alleviate some of the problems encountered in conventional aerosol delivery, due to their ability to (i) serve as a solubilization matrix for poorly soluble agents; (ii) act as a pulmonary sustained release reservoirs, and (iii) facilitate intracellular delivery of drugs, specifically to alveolar macrophages. Consequently, liposomes may provide a means to (i) prevent local irritation of lung tissue and reduce pulmonary toxicity, (ii) prolong local therapeutic drug levels, and (iii) generate high intracellular drug concentrations, e.g. in infected alveolar macrophages. Drugs that have been considered for pulmonary delivery via liposomes include anticancer drugs (antimicrobials, peptides, enzymes, antiasthmatic and antiallergic compounds as well as cromolyn sodium). Also immunomodulators, immunosuppressive agents, antiviral and antimycobacterial agents as well as gene constructs have been considered.

It is an object of the present invention to provide a drug delivery system for pulmonary delivery of active agents associated with liposomes or other such particulate carriers. Another object of the invention is to provide a tightly compacted solid medicament stock which is suitable for the generation of inhalable particles containing active agents.

The drug delivery system of the present invention comprises a tightly compacted solid medicament stock having an essentially isotropic solid state structure comprising an active agent and which stock is suitable for the generation of inhalable particles containing said active agents, wherein the tightly compacted solid medicament stock comprises at least one active agent which is associated with the particulate carrier, such as liposomes. The present invention is inter alia based on the unexpected finding that liposomes can withstand the high pressures which must be applied to form the tightly compacted solid medicament stock containing the liposomes. It is further surprising that the carriers, especially liposomes, which were loaded with an active agent or associated with an active agent, are not emptied or fully destroyed, when a powder containing e.g. liposomes is generated by, for example, abrading methods performed with a device as disclosed in WO 93/24165 (which is incorporated by reference) from this tightly compacted medicament stock. Preferably, pressures up to 500 MPa are applied according to WO 94/14490 (which is also incorporated by reference).

Surprisingly, the agent-associating liposomes survive both the compacting process (as in WO 97/14490) and the abrasion process (as in WO 93/24165) quite intact and apparently unchanged.

According to the invention it is preferred that the active agent which is associated with the carriers is arranged in and/or arranged on said carrier. Preferably the active agent is e.g. incorporated in liposomes or liposomes are loaded with the active agent, i.e. the active agent is encapsulated by the liposomes. However, in loading processes it may well occur that the active agent is also incorporated in the outer membrane enclosing the liposome, or even on the outer surface of the liposome membrane, so that the active agent is in this case not only released from the inner part of the liposome, but also from the membrane or shell of the liposome.

The nature of the particulate carrier, especially the liposomes is basically not critical for the drug delivery system of the present invention. All kinds of liposomes including negatively charged, neutral and cationic liposomes and lipid complexes as described in WO 96/27393 can be pressed to a tightly compacted medicament stock of the invention. Liposomes as described in our earlier application, now EP 0 639 373, can be employed in practising this invention.

Preferably, the liposomes which are associated with at least one active agent, are combined with at least one auxiliary material, such as a filler material. Preferably, the auxiliary material is a pharmaceutically acceptable filler material e.g. selected from physiologically acceptable sugars or salts. Particularly preferred are auxiliary materials selected from lactose, trehalose, glucose, mannit, sodium chloride and combinations thereof. It is preferred to use lactose as the auxiliary material in a ratio of at least one hundred parts by weight lactose to one part by weight liposome. In a preferred embodiment, the medicament stock of the drug delivery system of the invention is compacted by isostatic pressing with pressures of from 50 to 500 MPa.

The at least one active agent can basically be any agent which shows pharmaceutical or biological effects, ranging from for example, small molecules to artificial human chromosomes, as long as they can be loaded or associated with liposomes. In particular the at least one active agent is selected from the group comprising
- β₂-sympathomimetics having a short duration of effect such as salbutamol, terbutalin, fenoterol, bambuterol,
- β₂-sympathomimetics having a long duration of effect such as salmeterol, formoterol,
- corticosteroids for inhalative purposes such as budesonide, beclomethason, fluticasone,
- anti-cholinergic agents such as ipatropium bromide, oxitropium bromide,
- nonsteroidal anti-allergic agents such as DSCG, nedocromile,
- anti-inflammatory, especially antibiotic and/or antiseptic agents, such as povidone-iodine
and combinations thereof.

Furthermore, the at least one active agent in the medicament stock of the drug delivery system of the present invention may be selected from
- β-lactam antibiotics such as penicillins, cephalosporines, imipinem; aminoglycosides such as tobramycin, gentamycin; inhibitors of gyrase such as ofloxacin, ciprofloxacin.
- anti-viral agents such as ganciclovir. azidothymidin,
- anti-mycotic agents such as polyeme, azole,
- vaccines against measles, German measles, diphtheria, pertussis, polio and the like,
- vaccines consisting essentially of viral or bacterial components,
- vaccines containing DNA coding for the generation of specific antigens,
- opioids for alleviation and therapy of pain such as morphine, oxycodone, hydromorphone, buprenorphine, fentanyl, alfentanyl, sulfentanyl and the like.
- anti-infective agents such as oligo/polyribo- and/or oligo/polydesoxyribonucleic acids,
- peptides, polypeptides such as insulin, low and high molecular heparin,
- hormones such as growth factors, hormones of the thyroid gland, sex hormones, calcitonin,
and combinations thereof.

Unless extra precautions are taken, the invention may be less suitable for hygroscopic agents, agents which are easily oxidized, and some agents unstable when exposed to light. However, it is often possible to add suitable protective substances to the liposome membrane forming amphiphilics, to the interior substances of the liposome, and/or to the auxiliary or carrier substances, to sufficiently protect and stabilize even such sensitive agents for use with this invention.

In order to generate the particulate material from the tightly compacted medicament stock of the invention, any suitable method for the generation of powder from a solid precursor may be employed. Particularly preferred are methods such as micronization or abrading methods especially those disclosed in EP 0 407 028 (incorporated by reference) as well as WO 94/14490 (incorporated by reference).

The particles which are generated from the tightly compacted medicament stock preferably have particle sizes of from 0.1 to 50 µm, more preferred of from 1 to 8 µm when used in pulmonar applications. The respective particle sizes are preferably from 1 to 15 µm when used for nasal applications.

The method for the preparation of the medicament stock to be used in the drug delivery system according to the invention comprises the steps of preparation of the particulate carrier, especially the liposomes, and loading in a per se known manner. Optionally, the loaded carrier particles are mixed with at least one auxiliary material and are subsequently formed to a shaped body by isostatic pressing. Preferably, the pressure applied is in the range of from 50 to 500 MPa. The shaped body is preferably shaped to be used with the device as disclosed in WO 93/24165. This is especially a ring tablet as disclosed in WO 94/14490.

Where the carrier is a liposome material, a dried lipid film, for example, of phosphatidylcholine and phosphatidylglycerol may e.g. be dispersed in an aqueous solution, preferably in a physiological solution. The respective active agent is present in the same solution, as is (optionally) the auxiliary material. The dispersion is treated by intimately mixing the ingredients, for example, by shaking. Then the dispersion undergoes one or more freeze-thaw cycles and is emulsified, preferably by extrusion through a membrane. The freeze-thaw and emulsification cycles are repeated several times. In order to remove unencapsulated active agent. methods for separation of liposomes and active drug are employed. These methods are known to the skilled person and are selected depending on the size of the active agent to be encapsulated. If, for example, a low molecular weight active agent has to be separated from the liposome dispersion this may be performed by dialyzing the mixture. The final dispersion is further processed, for example, subsequently by freezing and freeze-drying. The solid material, preferably prepared by lyophilization is titurated to give a coarse liposome granulate. This granulate is mixed with the auxiliary material and subsequently compressed at pressures between 50 and 500 MPa. Solid, non-brittle tablets having a smooth surface are obtained by such procedures.

The tightly compacted solid medicament stock to be used in the drug delivery system of the invention is useful for the generation of particles which can be administered by insufflation and/or inhalation. The tightly compacted solid medicament can be used in conventional therapy of diseases and disorders of the respiratory tract as well as diseases and disorders of organs. The medicament stock of the invention can also be used in gene therapy and/or vaccination.

Gene therapy is emerging as a clinically viable therapeutic regimen for genetic, neoplastic and infectious diseases. A prominent example is cystic fibrosis, a genetic disease resulting from mutation of the cystic fibrosis transmembrane conductance regulator (CFTR) gene. Because of this pulmonary delivery of liposomal carriers of gene constructs has received much attention recently. However, it has been shown experimentally, that repeated cycling of DNA with or without liposomes, through a conventional nebulizer is detrimental for DNA and leads to complete degradation within minutes from the onset of nebulization. In addition to these, and in view of other general liposome-related stability considerations, gene therapy requires relatively concentrated doses of DNA complexes or encapsulated within liposomes to be delivered. Concentrated solutions of DNA/lipid complexes tend, however, to aggregate and precipitate, thus making nebulization of aqueous dispersions impractical. The drug delivery system of the present invention using the tightly compacted medicament stock of the invention, however, is able to overcome the drawbacks of other DNA/liposome administration methods as known in the prior art. Other gene therapy objectives may be the curing of factor IX and α₁-antitrypsin gene deficiencies as well as treatment of hemophilia which is related to deficiencies of factor VIII production or production of other important proteins in the blood clotting cascade. Of course, it is to be understood that the above-mentioned gene therapy targets are only mentioned for illustrative purposes. They are not intended to limit the scope of the invention.

Also vaccination can be effected by delivery of respective structures or substances with the drug delivery system of the present invention. In this case the conventional vehicles for inducing an immunoresponse can be administered by carriers such as liposomes which are present in a tightly compacted medicament stock of the invention.

### Example I

A dry lipid film consisting of 5.76 grams of phosphatidylcholine and 0.64 grams of phosphatidylglycerol was dispersed in 160 ml of phosphate-buffered saline (PBS) containing 3.8 grams of fluorescein isothiocyanate-dextran (FITC-dextran) and 28.8 grams of alpha-lactose. The dispersion was shaken manually for 2 hours, then underwent for freeze-thaw cycles and was emulsified by extrusion through 100 nm pore-size membranes (Poretic) for 10 min at no more than 3,000 psi using an EmulsiFex-C5 (Avestin) homogenizer. The freeze-thaw and extrusion cycle was repeated three times. In order to remove unencapsulated FITC-dextran, the liposome dispersion was dialyzed against a lactose solution (1,800 grams of alpha-lactose/10 L PBS) using a hollow-fiber cartridge with a molecular weight cutoff of 18,000 at a flow rate of 8 - 10 ml/min. After three dialysis cycles, the fluorescence concentration remained constant at approximately 50% of the original concentration as measured with a Hitachi F-2000 fluorescence spectrophotometer. The final dispersion was frozen in a mixture of dry ice and ethanol and transferred to a lyophilizer (Edwars Supermodulyo). The frozen preparation was freeze-dried at -40°C and 0.07 mbar for 48 hrs. followed by secondary drying at 25°C for 4 hrs.. The vacuum was replaced with dry nitrogen. The yield was 41.7 grams. The lyophilized cake was triturated to give a free flowing, coarse liposome granulate. This granulate was mixed at a 1:10 Ratio w/w with lactose and compressed isostatically at 150 MPa for approximately 60 sec.. Solid, non-brittle tablets with a smooth surface were produced, mounted onto the plastic holder of the device according to WO 93/24165 and stored at room temperature in a plastic container. Tablets were actuated 20 times. The total mass shaved from the tablet surface per actuation and the FITC-dextran content per actuation were determined. A total delivered mass of 9.66 +/- 0.99 mg was found, containing 208 +/-63 F.U. of FITC-dextran.

### Example II

The above procedure was repeated using liposomes made in accordance with EP 0 639 373, as follows:

In a 1000 ml glass flask, provided with glass beads for increased surface, 51.9 mg cholesterol and 213 mg hydrogenated soy bean lecithine were dissolved in a sufficient amount of a mixture of methanol and chloroform in a 2:1 ratio. The solvent was then evaporated under a vacuum until a film was formed on the inner surface of the flask and on the glass beads.

2.4 g PVP iodine (containing about 10% available iodine) were separately dissolved in 12 ml water.

Again in a separate vessel, 8.77 g sodium chloride and 1.78 g Na₂HPO₄·2H₂O were dissolved in 400 ml water. Further water was added up to a total volume of 980 ml, and then, approximately 12 ml IN hydrochloric acid were added to adjust pH to 7.4. This solution was then topped up with water to exactly 1000 ml.

In a fourth vessel, 900 mg saccharose and 57 mg disodium succinate were dissolved in 12 ml water.

The PVP iodine solution was then added to the lipid film in the flask and the mixture was shaken until the film dissolved. This produced liposome formation from the hydrated lipids in the flask. The product was centrifuged and the supernatant liquid was discarded. The saccarose solution was added ad 12 ml and the product was again centrifuged. Afterwards the supernatant liquid was again discarded. At this stage, a further washing step, using the saccharose solution or the sodium chloride buffer solution could be used.

After the last centrifugation step and discarding of the supernatant, sodium chloride buffer solution was added ad 12 ml, and the liposomes were homogenously distributed therein. The product was then distributed into vials each containing 2 ml liposome dispersion, and the vials were then subjected to a freeze-drying step.

After the freeze-drying, each vial comprised about 40 mg solids.

The solid liposomes were then compacted, with lactose, as described in Example I.

The ring tablets were used with the abrading device of WO 93/24165 and delivered a total of approximately 10 mg of powder upon each actuation. The PVP-iodine content in the powder corresponded to the theoretical (calculated) value, within the experimental error margin.

## Claims

1. A drug delivery system comprising a tightly compacted solid medicament stock having an essentially isotropic solid state structure comprising an active agent and which stock is suitable *for* the generation of inhalable particles containing said active agent, wherein the tightly compacted solid medicament stock comprises at least one active agent which is associated with liposomes or a similar particulate carrier.

2. The drug delivery system of claim 1, wherein the at least one active agent comprised in the tightly compacted medicament stock is encapsulated in and/or associated with the membranes of said liposomes.

3. The drug delivery system of claim 1 and/or 2, wherein said tightly compacted solid medicament stock contains said liposomes together with auxiliary material such as filler materials.

4. The drug delivery system of any one of the claims 1 to 3, wherein said at least one active agent comprised in the tightly compacted solid medicament stock is selected from the group comprising
- β₂-sympathomimetics having a short duration of effect such as salbutamol, terbutalin, fenoterol, bambuterol,
- β₂-sympathomimetics having a long duration of effect such as salmeterol, formoterol,
- corticosteroids for inhalative purposes such as budesonide, beclomethason, fluticasone,
- anti-cholinergic agents such as ipatropium bromide, oxitropium bromide,
- nonsteroidal anti-allergic agents such as DSCG, nedocromile,
- anti-inflammatory, especially antiobiotic and/or antiseptic agents such as povidone-iodine
and combinations thereof.

5. The drug delivery system of any one of the claims 1 to 3, wherein said at least one active agent comprised in the tightly compacted solid medicament stock is selected from the group comprising
- β-lactam antibiotics such as penicillins, cephalosporines, imipinem; aminoglycosides such as tobramycin, gentamycin; inhibitors of gyrase such as ofloxacin, ciprofloxacin,
- anti-viral agents such as ganciclovir, azidothymidin,
- anti-mycotic agents such as polyeme, azole,
- vaccines against measles, German measles, diphtheria, pertussis, polio and the like,
- vaccines consisting essentially of viral or bacterial components,
- vaccines containing DNA coding for the generation of specific antigens,
- opioids for alleviation and therapy of pain such as morphine, oxycodone, hydromorphone, buprenorphine, fentanyl, alfentanyl, sulfentanyl and the like,
- anti-infective agents such as oligo/polyribo- and/or oligo/polydesoxyribonucleic acids,
- peptides, polypeptides such as insulin, low and high molecular heparin,
- hormones such as growth factors, hormones of the thyroid gland, sex hormones, calcitonin.
and combinations thereof.

6. The drug delivery system of any one of the claims 1 to 5, wherein the tightly compacted solid medicament stock has been compacted by isostatic pressing with pressures of from 50 to 500 MPa.

7. The drug delivery system of any one of the claims 1 to 6, wherein the auxiliary material in the tightly compacted solid medicament stock is selected from lactose, trehalose, glucose, mannit sodium chloride and combinations thereof.

8. The drug delivery system of any one of the claims 1 to 7, wherein the liposomes are mixed with lactose in a ratio of at least 100 parts by weight lactose to 1 part by weight liposome.

9. The drug delivery system of any one of the claims 1 to 5, wherein the tightly compacted medicament stock is subjected to suitable processes to generate particles such as by micronisation or abrading.

10. The drug delivery system of claim 9, wherein the generated particles have particle sizes ranging from 0.1 to 50 µm, in particular from 1 to 8 µm when used in pulmonal applications, and from 1 to 15 µm when used for nasal applications.

11. The drug delivery system of claim 1 wherein the particulate carrier is selected from microspheres, nanoparticles, Large Porous Particles, artificial virus envelopes and similar pharmaceutically acceptable materials.

12. A method for the preparation of the tightly compacted solid medicament stock of the drug delivery system of any one of the claims 1 to 11, wherein the liposomes are prepared or loaded in a known manner, are optionally mixed with at least one auxiliary material, and are subsequently formed to a shaped body by isostatic pressing.

13. The method of claim 12, wherein the isostatic pressing is performed with pressures of from 50 to 500 MPa.

14. Use of a tightly compacted solid medicament stock in a drug delivery system of any one of the claims 1 to 11 for the generation of particles which can be administered by insufflation and/or inhalation, in the therapy of diseases and disorders of the respiratory tract as well as diseases and disorders of organs, gene therapy and/or vaccination.

## Revendications

1. Système de distribution de médicaments comprenant un stock de médicaments solides fortement compactés, ayant une structure à l'état solide essentiellement isotrope comprenant un agent actif et ledit stock étant approprié à la génération de particules pouvant être inhalées contenant ledit agent actif, dans lequel ledit stock de médicaments solides fortement compactés comprend au moins un agent actif qui est associé à des liposomes ou un support particulaire similaire.

2. Système de distribution de médicament selon la revendication 1, dans lequel au moins un agent actif contenu dans le stock de médicaments fortement compactés est encapsulé dans et/ou associé avec les membranes desdits liposomes.

3. Système de distribution de médicaments selon la revendication 1 et/ou 2, dans lequel ledit stock de médicaments solides fortement compactés contient lesdits liposomes en compagnie de substances auxiliaires comme les charges.

4. Système de distribution de médicaments selon l'une quelconque des revendications 1 à 3, dans lequel ledit au moins un agent actif contenu dans le stock de médicaments solides fortement compactés est choisi parmi le groupe comprenant les :
- β₂-sympathomimétiques ayant un effet de courte durée comme le salbutamol, la terbutaline, le fénotérol, le bambutérol,
- β₂-sympathomimétiques ayant un effet de longue durée comme le salmétérol, le formotérol,
- corticostéroïdes à des fins d'inhalation comme le budésonide, la béclométhasone, la fluticasone,
- agents anti-cholinergiques comme le bromure d'ipatropium, le bromure d'oxitropium,
- agents anti-allergiques non stéroïdiens comme le DSCG, le nédocromile,
- agents anti-inflammatoires, particulièrement antibiotiques et/ou antiseptiques, comme la povidone-iode,
et combinaisons de ceux-ci.

5. Système de distribution de médicaments selon l'une quelconque des revendications 1 à 3, dans lequel ledit au moins un agent actif contenu dans le stock de médicaments solides fortement compactés est choisi parmi le groupe comprenant les :
- antibiotiques β-lactame comme les pénicillines, les céphalosporines, l'imipinem ; les aminoglycosides comme la tobramycine, la gentamycine ; les inhibiteurs de gyrase comme l'ofloxacine, la ciprofloxacine,
- agents anti-viraux comme le ganciclovir, l'azidothymidine,
- agents anti-mycosiques comme le polyème, l'azole,
- vaccins contre la rougeole, la rubéole, la diphtérie, la coqueluche, la poliomyélite et similaire,
- vaccins composés essentiellement de composants viraux ou bactériens,
- vaccins contenant de l'ADN codant pour la génération d'antigènes spécifiques,
- opioïdes pour le soulagement et le traitement de la douleur comme la morphine, l'oxycodone, l'hydromorphone, la buprénorphine, le fentanyl, l'alfentanyl, le sulfentanyl et similaire,
- agents anti-infectieux comme les acides oligo/polyribo- et/ou oligo/polydésoxyribonucléiques,
- peptides, polypeptides, comme l'insuline, l'héparine à faible poids moléculaire et poids moléculaire élevé,
- hormones comme les facteurs de croissance, les hormones de la glande thyroïde, les hormones sexuelles, la calcitonine,
et combinaisons de ceux-ci.

6. Système de distribution de médicaments selon l'une quelconque des revendications 1 à 5, dans lequel le stock de médicaments solides fortement compactés a été compacté par pression isostatique à des pressions allant de 50 à 500 MPa.

7. Système de distribution de médicaments selon l'une quelconque des revendications 1 à 6, dans lequel l'auxiliaire dans le stock de médicaments solides fortement compactés est choisi parmi le lactose, le tréhalose, le glucose, le chlorure de mannite sodique et des combinaisons de ceux-ci.

8. Système de distribution de médicaments selon l'une quelconque des revendications 1 à 7, dans lequel les liposomes sont mélangés avec le lactose dans un rapport d'au moins 100 parties en poids de lactose sur 1 partie en poids de liposome.

9. Système de distribution de médicaments selon l'une quelconque des revendications 1 à 5, dans lequel le stock de médicaments fortement compactés est soumis à des procédés adéquats pour générer des particules par exemple par micronisation ou abrasion.

10. Système de distribution de médicaments selon la .revendication 9, dans lequel les particules générées ont des tailles de particules allant de 0,1 à 50 µm, en particulier de 1 à 8 µm quand on les utilise dans des applications pulmonaires et de 1 à 15 µm quand on les utilise pour des applications nasales.

11. Système de distribution de médicaments selon la revendication 1, dans lequel le support particulaire est choisi parmi les microsphères, les nanoparticules, les grosses particules poreuses (LPP), les enveloppes de virus artificiels et substances similaires pharmaceutiquement acceptables.

12. Procédé de préparation du stock de médicaments solides fortement compactés du système de distribution de médicaments selon l'une quelconque des revendications 1 à 11, dans lequel les liposomes sont préparés ou chargés d'une manière connue, sont éventuellement mélangés avec au moins une substance auxiliaire et sont ultérieurement façonnés en un corps formé par pression isostatique.

13. Procédé de préparation selon la revendication 12, dans lequel la pression isostatique est réalisée à des pressions allant de 50 à 500 MPa.

14. Utilisation d'un stock de médicaments solides fortement compactés dans un système de distribution de médicaments selon l'une quelconque des revendications 1 à 11, pour la génération de particules qui peuvent être administrés par insufflation et/ou inhalation, dans la thérapie des maladies et troubles du tractus respiratoire ainsi que les maladies et troubles des organes, la thérapie génique et/ou la vaccination.

## Patentansprüche

1. Wirkstoffapplikationssystem, das einen hochverdichteten festen Arzneimittelvorrat umfasst, welcher eine im wesentlichen isotrope Festkörperstruktur aufweist, und das einen Wirkstoff umfasst, und der Vorrat für die Erzeugung von inhalierbaren Partikeln geeignet ist, welche den Wirkstoff enthalten, wobei der hochverdichtete feste Arzneimittelvorrat wenigstens einen Wirkstoff umfasst, welcher mit den Liposomen oder einem ähnlichen partikulären Träger assoziiert ist.

2. Wirkstoffapplikationssystem nach Anspruch 1, wobei der wenigstens eine Wirkstoff, welcher in dem hochverdichteten Arzneimittelvorrat umfasst ist, in den Membranen der Liposomen eingekapselt und/oder mit diesen assoziiert ist.

3. Wirkstoffapplikationssystem nach Anspruch 1 und/oder 2, wobei der hochverdichtete feste Arzneimittelvorrat die Liposomen zusammen mit Hilfsmaterial wie etwa Füllmaterialien enthält.

4. Wirkstoffapplikationssystem nach einem der Ansprüche 1 bis 3, wobei der wenigstens eine Wirkstoff, welcher in dem hochverdichteten festen Arzneimittelvorrat enthalten ist, ausgewählt ist aus der Gruppe umfassend
- β₂-Sympathomimetika, die eine kurze Wirkungsdauer aufweisen, wie etwa Salbutamol, Terbutalin, Fenoterol, Bambuterol,
- β₂-Sympathomimetika, die eine lange Wirkungsdauer aufweisen, wie etwa Salmeterol, Formoterol,
- Corticosteroide für Inhalationszwecke wie etwa Budesonid, Beclometason, Fluticason,
- Anticholinergika wie etwa Ipatropiumbromid, Oxitropiumbromid,
- nicht-steroidale Antiallergika wie etwa DSCG, Nedocromil,
- entzündungshemmende, insbesondere antibiotische und/oder antiseptische Wirkstoffe, wie etwa Povidon-Jod
und Kombinationen davon.

5. Wirkstoffapplikationssystem nach einem der Ansprüche 1 bis 3, wobei der wenigstens eine Wirkstoff, welcher in dem hochverdichteten festen Arzneimittelvorrat umfasst ist, ausgewählt ist aus der Gruppe umfassend
- β-Lactam-Antibiotika wie etwa Penicilline, Cephalosporine, Imipenem; Aminoglykoside wie etwa Tobramycin, Gentamycin; Gyrasehemmer wie etwa Ofloxacin, Ciprofloxazin,
- antivirale Wirkstoffe wie etwa Ganciclovir, Azidothymidin,
- Antimycotika wie etwa Polyem, Azol,
- Impfstoffe gegen Masern, Röteln, Diphtherie, Keuchhusten, Polio und ähnliches,
- Impfstoffe, die im wesentlichen aus viralen oder bakteriellen Komponenten bestehen,
- Impfstoffe, die eine DNA-Codierung für die Generierung von spezifischen Antigenen enthalten,
- Opioide zur Linderung und Therapie von Schmerzen wie etwa Morphin, Oxycodon, Hydromorphon, Buprenorphin, Fentanyl, Alfentanil, Sulfentanyl und ähnliches,
- Antiinfektiva wie etwa Oligo/Polyribo- und/oder Oligo/Poly-desoxyribonucleinsäuren,
- Peptide, Polypeptide wie etwa Insulin, nieder- und hochmolekulares Heparin,
- Hormone wie etwa Wachstumsfaktoren, Hormone der Schilddrüse, Sexualhormone, Calcitonin
und Kombinationen davon.

6. Wirkstoffapplikationssystem nach einem der Ansprüche 1 bis 5, wobei der hochverdichtete feste Arzneimittelvorrat durch isostatisches Pressen mit Drücken von 50 bis 500 MPa verdichtet wurde.

7. Wirkstoffapplikationssystem nach einem der Ansprüche 1 bis 6, wobei das Hilfsmaterial in dem hochverdichteten festen Arzneimittelvorrat ausgewählt ist aus Laktose, Trehalose, Glucose, Mannit, Natriumchlorid und Kombinationen davon.

8. Wirkstoffapplikationssystem nach einem der Ansprüche 1 bis 7, wobei die Liposomen mit Laktose in einem Verhältnis von wenigstens 100 Gewichtsanteilen Laktose zu 1 Gewichtsanteil Liposom vermischt werden.

9. Wirkstoffapplikationssystem nach einem der Ansprüche 1 bis 5, wobei der hochverdichtete Arzneimittelvorrat geeigneten Verfahren zur Erzeugung von Partikeln unterzogen wird, wie etwa Mikronisation oder Abschleifen.

10. Wirkstoffapplikationssystem nach Anspruch 9, wobei die erzeugten Partikel Partikelgrößen im Bereich von 0,1 bis 50 µm, insbesondere von 1 bis 8 µm bei Verwendung bei pulmonalen Anwendungen und von 1 bis 15 µm bei Verwendung bei nasalen Anwendungen aufweisen.

11. Wirkstoffapplikationssystem nach Anspruch 1, wobei der partikuläre Träger ausgewählt ist aus Mikrosphären, Nanopartikeln, Large Porous Particles, künstlich hergestellten Virenhüllen und ähnlichen pharmazeutisch zulässigen Materialien.

12. Verfahren zur Herstellung des hochverdichteten festen Arzneimittelvorrats des Wirkstoffapplikationssystems nach einem der Ansprüche 1 bis 11, wobei die Liposomen in bekannter Weise hergestellt und beladen werden, gegebenenfalls mit wenigstens einem Hilfsmaterial vermischt werden und nachfolgend durch isostatisches Pressen zu einem Formkörper geformt werden.

13. Verfahren nach Anspruch 12, wobei das isostatische Pressen mit Drücken von 50 bis 500 MPa durchgeführt wird.

14. Verwendung eines hochverdichteten festen Arzneimittelvorrats in einem Wirkstoffapplikationssystem nach einem der Ansprüche 1 bis 11 zur Erzeugung von Partikeln, welche durch Einblasen und/oder Inhalieren bei der Therapie von Krankheiten und Störungen der Atemwege sowie Krankheiten und Störungen der Organe, bei der Gentherapie und/oder der Impfung verabreicht werden können.
